# EUROPEAN PATENT APPLICATION

(11) **EP 4 847 040 A2**
(43) Date of publication of application: **02.12.2026**
(21) Application number: 26197149.3
(22) Date of filing: 22.01.2021
(51) Int. Cl.: A61K 39/395

(54) **METHODS OF STIMULATING BONE GROWTH WITH ABALOPARATIDE AND DENOSUMAB**

(30) Priority: 24.01.2020 US 202062965214 P
(62) Document number(s) of the earlier application(s) in accordance with Art. 76 EPC: 21702314.2 / 4 110 371
(71) Applicant: Radius Health, Inc., Boston MA 02210 (US)
(72) Inventor: HATTERSLEY, Gary, Stow, 01775 (US); YATES, John, Boulder, 80304 (US); MITLAK, Bruce, Waltham, 02451 (US)
(74) Representative: Carpmaels & Ransford LLP

(57) **Abstract**

Provided herein are methods for increasing bone mineral density, treating osteoporosis and the like, that include administration of abaloparatide in combination with denosumab.

## Description

### RELATED APPLICATIONS

The present application claims priority to US Provisional Application No. 62/965,214, filed January 24, 2020, which is incorporated herein by this reference in its entirety, including drawings.

### SEQUENCE LISTING

The instant application contains a Sequence Listing which has been submitted electronically in ASCII format and is hereby incorporated by reference in its entirety. Said ASCII copy, created on January 22, 2021, is named SL_R105231_1090WO.txt and is 1,120 bytes in size.

### TECHNICAL FIELD

Provided herein are compositions, devices, methods and processes related to the administration of abaloparatide in combination with denosumab.

### BACKGROUND

Parathyroid hormone-related protein ("PTHrP") is a 139 to 173 amino acid-protein. PTHrP, especially the C-terminal 1-36 secretory product and certain analogs, are known to be useful for the treatment of osteoporosis and related disorders by stimulating bone formation to improve bone mineral density (BMD). PTHrP analogues having excellent pharmacological properties and parenteral storage stable compositions thereof are described in Int. Publ. No. WO 2008/063279, and solid microstructured transdermal system ("sMTS") microprojection arrays thereof are described in Int. Publ. No. WO 2012/145665 and Int. Publ. No. WO 2013/082427, the entire contents of which are hereby incorporated by reference.

Preclinical studies have demonstrated marked bone anabolic activity of the PTHrP(1-34) analog abaloparatide with complete reversal of bone loss in ovariectomy-induced osteopenia animal models. Phase 2 and 3 clinical studies have demonstrated that 100 µg and 150 µg doses of abaloparatide transdermal (TD) patches and the 80 µg dose of abaloparatide subcutaneous (SC) injection demonstrate consistent increases in BMD in both lumbar spine and hip. All doses of abaloparatide TD and abaloparatide SC were well tolerated, with no reported treatment-related serious adverse events. The increases in BMD seen are substantially faster and greater than those seen with either cathepsin K inhibitor drugs or PTH-like drugs such as teriparatide. Abaloparatide has a lower potential to induce hypercalcemia at pharmacologically effective doses compared to teriparatide and has greater stability at room temperature, allowing greater storage convenience.

Some patients on denosumab treatment for osteoporosis remain at high risk for fracture. These include women who sustain incident fractures on denosumab and those who have declining BMD or persistently low BMD, despite treatment. There are few options available for these patients. Denosumab withdrawal is associated with dramatic increased bone remodeling, rapid prominent bone loss, and multiple vertebral fractures (Cummings JBMR 2017 Feb; 33(2):190-198). Switching from denosumab to teriparatide is associated with substantial BMD loss in the hip and femoral neck. After 2 years of denosumab treatment, when women are switched to teriparatide, total hip BMD remains below the mean BMD (at end of denosumab treatment) over the entire 2 years of teriparatide treatment (Leder, Lancet. 2015 Sep 19;386(9999)).

There is a need for efficacious and safe treatments which surpass current standard of care. Furthermore, there is a need for osteoporosis therapy that could limit or avoid subcutaneous injections, or provide greater room temperature stability, or provide substantially greater increases in BMD, any of which could enhance patient compliance and/or convenience as well as the effectiveness of treatment. There is also a need for osteoporosis therapy that is capable of reducing adverse events typically observed with the standard of care.

### SUMMARY OF THE INVENTION

In one aspect the present invention relates to combinations of PTHrP(1-34) analogues (e.g., abaloparatide) and denosumab and methods of treating osteoporosis, osteopenia, fractured bones and osteoarthritis in a subject in need of treatment. The method comprises administering to the subject an effective amount of a combination of a PTHrP(1-34) analog (e.g., abaloparatide) and denosumab to a subject in need thereof. In a particular embodiment, administering an effective amount of a combination of a PTHrP analog and denosumab comprises coadministering to said subject a first amount of a PTHrP analog or a pharmaceutically acceptable salt thereof and a second amount of denosumab, wherein the first and second amounts together comprise a therapeutically effective amount.

The preferred method of the invention comprises administering to the subject abaloparatide and denosumab. In one embodiment, the method includes administering the abaloparatide concomitantly, e.g., administering abaloparatide daily while administering denosumab every 6 months. In another embodiment, abaloparatide therapy begins after denosumab has been administered for 6 months, a year, 18 months, or two years prior. In some embodiments, denosumab therapy continues and is ongoing through the abaloparatide therapy. In other embodiments, the abaloparatide therapy begins after or before denosumab therapy.

In certain embodiments, the combinations and methods described herein are useful for the treatment of osteoporosis. In some embodiments, the combinations and methods described herein are useful for the treatment of postmenopausal osteoporosis. In certain embodiments, the combinations and methods described herein are useful for the treatment of glucocorticoid induced osteoporosis in men or women. In certain embodiments, the methods of treating osteoporosis described herein can be applied to a patient or patient population characterized as being at an elevated risk for bone fracture.

In certain embodiments, a method of treating fractures or accelerating fracture healing in a subject in need thereof is provided comprising the administration of abaloparatide and denosumab.

In some embodiments, a method of increasing bone mineral density in a subject in need thereof is provided comprising the administration of abaloparatide in combination with denosumab.

In some embodiments, the method includes administering abaloparatide in an amount of 80 mcg daily subcutaneously or 300 mcg daily transdermally. In some embodiments, the method includes administering denosumab in an amount of 60 mg administered subcutaneously every six months. The abaloparatide can be administered concomitantly or sequentially before or after administration of denosumab.

In some embodiments, the total hip BMD is increased at least 5%, 10%, 15% or 20% at 18 months. In some embodiments, the total hip BMD is increased at least 1%, 3%, 5%, 10%, 15% or 20% at 18 months. In some embodiments, the total hip BMD is increased at least 1%, e.g. between about 1% and about 5% at 18 months.

In some embodiments, the total spine BMD is increased at least 12%, 15%, or 20% at 18 months in subjects where abaloparatide is administered with ongoing treatment with denosumab.

In some embodiments, the total spine BMD is increased at least 4%, 5%, 10%, or 15% at 18 months in subjects where abaloparatide is administered in subjects that have already received at least 2 years of treatment with denosumab.

In some embodiments, the subject is a subject at high risk for a fracture despite denosumab treatment, as indicated by declining or persistently low bone mass density.

In some embodiments, the subject has had denosumab therapy, followed by teriparatide therapy, and a total hip BMD below the baseline at the start of teriparatide therapy. In some embodiments the subject's osteoporosis or low BMD is refractory to treatment with denosumab, or with teriparatide, or with both.

### BRIEF DESCRIPTION OF THE DRAWINGS

**FIG. 1** is an illustration of a circular patch.
**FIG. 2** is a side view with dimensions of the microstructures of the circular patch.

### DETAILED DESCRIPTION OF THE INVENTION

Osteoporosis results from an imbalance of bone remodeling with an increase in the rate of bone resorption coupled with reduced new bone formation leading to net bone loss (Rosen CJ. N Engl J Med 2005;353:595-603.). This loss of bone is associated with a reduction in bone quality and integrity, and a marked increase in the risk of fractures. With regard to osteopenia or osteoporosis, it will not matter if the osteoporosis or risk of osteoporosis from which the subject suffers finds its roots in immobilization, age, low gonadal state (e.g. postmenopausal women, testosterone deficient males - including chemically-induced low gonadal -like states induced through use of aromatase inhibitors, anti-androgens, gonadotropin agonist/antagonists and the like), etidocritiological disorders (e.g., diabetes, adrenal insufficiency, Cushing's syndrome), malnutrition including vitamin D and/or calcium deficiency, rheumatoid arthritis, renal insufficiency, various cancers including myelomas and leukemias, certain inherited forms of osteoporosis and osteoporosis caused by concomitant administration of medicines known or suspected to cause bone loss (e.g., corticosteroids, peroxisome proliferator- activated receptor gamma (PPARgamma) agonists, prolonged bed rest or immobilization, thyroid medications, lithium therapy, anti-depressants, proton pump inhibitors, etc). Whatever the source, osteoporosis risk is most broadly identified by identifying at risk populations but more specifically can be identified by looking at individual risk factors including low bone mineral density and/or prior incidence of fracture in the individual in question.

Without wishing to be bound to any particular theory, it is believed that abaloparatide will be a better option than teriparatide in patients switching from denosumab, because it is less pro-resorptive than teriparatide. It is also believed that adding abaloparatide to ongoing denosumab may also be an excellent treatment option. Adding abaloparatide to continued denosumab treatment will allow bone formation to increase, without increasing bone resorption (modeling-based bone formation) and will produce substantial BMD increments in both spine and hip. And it is believed that in patients who still appear to be at high risk for fracture while receiving ongoing denosumab therapy, adding abaloparatide will increase BMD of the lumbar spine and total hip significantly more than continuing denosumab alone.

It is additionally believed that the abaloparatide and denosumab may be capable of significantly reducing adverse events that are typically observed with abaloparatide and denosumab individually (e.g. hypotensive effects, dizziness, palpitation and nausea side effects). Without wishing to be bound by any particular theory, it is believed that the combination of abaloparatide and denosumab will reduce adverse events by providing a favourable PTH profile since abaloparatide is capable of decreasing endogenous PTH levels while denosumab is capable of increasing endogenous PTH levels.

In one aspect, the present invention relates to combinations of PTHrP(1-34) analogues (e.g., abaloparatide) and denosumab and methods of treating osteopenia, osteoporosis, or osteoarthritis; for the treatment of bone fractures; to accelerate bone fracture healing; to increase bone mineral density. In another aspect the present invention relates to combinations of PTHrP(1-34) analogues (e.g., abaloparatide) and denosumab and methods to promote spinal fusion; or to enhance the rate of osteointegration of bone implants. The methods include administering to a subject, including a human, an effective amount of abaloparatide and concomitantly or sequentially (either before or after administration of abaloparatide) administering an effective amount of denosumab.

In one embodiment, the abaloparatide and the denosumab are in separate dosage forms provided separately. In another embodiment, separate dosage forms of abaloparatide and of denosumab are provided which are associated with one another. The term "associated with one another" as used herein means that the separate dosage forms are packaged together or otherwise attached to one another such that it is readily apparent that the separate dosage forms are intended to be sold and administered together (within less than 24 hours of one another, consecutively or simultaneously).

In addition, the methods of treating osteoporosis described herein may, for example, be applied to any patient at potentially increased risk of fracture wherein one or more characteristics that identify a patient as being at increased risk of fracture include, but are not limited to smoking, consumption of alcohol, use of glucocorticoids, use of tricyclic antidepressants, have an increased risk of falling, have asthma, have chronic liver disease, have rheumatoid arthritis, have type 2 diabetes, have endocrine problems, have familial history of fractures, and have poor nutrition or nutritional disorders.

It should be appreciated that the compositions, products, devices and methods described herein can be applied to at-risk populations or individuals. Because of the highly boneanabolic nature of the compositions and methods of this invention, for example, there is particular value in treating populations at especially high risk, including those with bone mineral density, as measured at one or more skeletal sites, of more than 1 standard deviation below the mean, or more than 2 standard deviations below the mean, or more than 2.5 standard deviations below the mean, or more than 3 standard deviations below the mean. Clinical endpoints for treatment of osteoporosis include reduced incidence of vertebral or non-vertebral fracture at end-of-treatment; bone mineral density of lumbar spine, hip, femoral neck and/or forearm (e.g., at or above the mean); and serum markers of bone formation and resorption (e.g., the amount of such markers). For example, serum PINP (procollagen type 1 N-terminal propeptide) and serum osteocalcin are markers of bone growth, while serum CTX (collagen type 1 cross-linked C-telopeptide) is a marker for bone resorption.

Alternatively, or in addition, the compositions and methods provided herein are of particular value for those who have had one or more previous bone fractures, particularly those who have suffered from one or more previous fragility fractures. A patient that has had one or more prior fractures may also present with a bone mineral density at or below the mean. For example, the patient may have bone mineral density at one or more sites that is at least 1 standard deviations below the mean, or at least 2 standard deviations below the mean, or at least 2.5 standard deviations below the mean or at least 3 standard deviations below the mean.

In certain therapeutic areas, the combinations and methods of this invention are useful for improving the healing process in people who have suffered from one or more fractures or breaks of one or more bones in their bodies, including either vertebral fractures or non-vertebral fractures (for example, hip or femur fractures). With regard to treatment of bone fracture or the acceleration of bone fracture healing, the fractures may be either high trauma or low trauma fractures, the latter including for example, fragility or osteoporotic fractures. In particular, osteoporotic fractures may occur at the hip, spine, wrist, or forearm, though they are not limited to these sites. People who have suffered a bone fracture may or may not suffer from concomitant low bone mineral density, but they can benefit from the increased rate of bone formation that by the use of the methods and combinations this invention can provide.

In some embodiments, the methods for improving the healing process in people who have suffered from one or more fractures or breaks of one or more bones in their bodies described herein can be applied to a patient with one or more vertebral fractures. In some embodiments, the methods for improving the healing process in people who have suffered from one or more fractures or breaks of one or more bones in their bodies described herein can be applied to a patient with one or more femoral fractures. In some embodiments, the methods for improving the healing process in people who have suffered from one or more fractures or breaks of one or more bones in their bodies described herein can be applied to a patient with one or more radial fractures. In some embodiments, the methods for improving the healing process in people who have suffered from one or more fractures or breaks of one or more bones in their bodies described herein can be applied to a patient with one or more fractures of the tibia and fibula.

The administration of the combinations and methods described herein can begin any time after a fracture is detected. In some embodiments, the administration is started no later than 6 months after a fracture has occurred or is detected. In certain embodiments, said administration is started no later than 3 months after a fracture has occurred or is detected. In some embodiments, said administration is started no later than 1 month after a fracture has occurred or is detected. In some embodiments, said administration is started no later than 2 weeks after a fracture has occurred or is detected. In certain embodiments, said administration is started no later than 1 week after a fracture has occurred or is detected. In certain embodiments, said administration is started after it has been clinically determined that fracture healing is delayed relative to normal fracture healing or that there is fracture non-union. In certain embodiments, said administration is started after it has been clinically determined that the patient is at high risk of delayed fracture healing or fracture non-union due to factors that may include the site or type of fracture, the adequacy of local blood supply and patient age. It is recommended that to most effectively utilize the method of treating people with one or more fractured bones is for treatment to begin soon after a fracture is detected. It should be appreciated that the duration of treatment is contingent upon a number of variables including the extent of the injury, the location of the injury, the rate and degree of recovery, the patient's overall bone health including bone mineral density at other anatomical sites, the discretion of the treating physician and more. Therefore, the treatment of fracture can vary from as little as one administration up to one year of treatment or even longer term treatment. In certain embodiments, the treatment period will be at least one week of once-daily administration of abaloparatide.

Improvement in the healing process is evidenced by an increase in fracture healing rate and/or quality of bone associated with the fractured site and/or patient-reported symptomatic outcomes including such indices of fracture healing such as reduced discomfort, increased flexibility and/or mobility and/or strength. Clinical endpoints for fracture healing include radiographic or functional evidence of healing, for example, by determining callus formation at the fracture site by a CT scan to determine healing (early/beginning callus formation) or healed (complete callus formation), fracture-site pain, and increased bone density.

The combinations and methods provided herein may also be used for the prevention and/or treatment of osteoarthritis. It is recognized that osteoarthritis is accompanied by the loss of cartilage, particularly at the joints. In some cases, the lost cartilage is replaced by bone or bony deposits. The combinations and methods of this invention described herein provide methods of treating people with agents that promote the bone remodeling process possibly including the increased production of cartilage and/or the diminution of bony deposits through acceleration of a normal bone remodeling process. Increasing the amount of cartilage in worn joints can have a beneficial effect on the individual measurable by numerous quality of life improvements including decreased pain and increased freedom of motion around the affected joint. Since the signs and symptoms of osteoarthritis are often different than osteoporosis, the treatment of osteoarthritis by the methods of this invention will take that into account. In particular, whereas the effect of an osteoporosis treatment can be readily ascertained by acute temporal effects on bone mineral density and reduction in fracture risk, the effect of treatment for osteoarthritis can be most readily detected via a patient reported reduction of symptoms. In this regard, the treatment of osteoarthritis can be started upon the observation of one or more symptoms of osteoarthritis and may be continued for a time sufficient for the diminution or elimination of one or more of the observed symptoms. Alternatively, the patient can have their treatment monitored by X-ray analysis of the affected joint(s) and the X-ray images interpreted by a qualified examiner in order to help determine if the treatment is having the desired effect. Due to the complexity of osteoarthritis and the ambiguity of correlating X-ray images with patient perception of pain or affected movement, the patient together with their medical practitioner will often decide together whether the treatment regimen is working or whether it should be adjusted.

The methods and combinations of the present administration can be administered for clinically appropriate periods. In some embodiments, clinically appropriate periods range from 1 month to 24 months. For example, clinically appropriate periods are 1 month, 3 months, 6 months, 9 months, 12 months, 15 months, 18 months, or 24 months. In some embodiments, a clinically appropriate period is until a clinically desired increase in BMD is achieved.

### Combination therapy: Abaloparatide and Denosumab

The sequence of native hPTHrP (1-34) is as follows: Ala Val Ser Glu His Gln Leu Leu His Asp Lys Gly Lys Ser Ile Gln Asp Leu Arg Arg Arg Phe Phe Leu His His Leu Ile Ala Glu Ile His Thr Ala (SEQ ID NO:1).

The preferred compound for use in the various embodiments of this invention is [Glu22,25, Leu23,28,31, Aib29, Lys26,30]hPTHrP(1-34)NH₂ (SEQ ID NO:2), also known as abaloparatide, or a salt thereof. Abaloparatide is a modified 34 amino acid N-terminus fragment of full length human PTHrP which is found in humans in three variants: PTHrP (1-139), PTHrP (1-141) and PTHrP (1-173). Abaloparatide, abaloparatide compositions, and methods of treating, e.g., osteoporosis, are described, e.g., in International Publication No. WO 2008/063279, US Patent No. 7,803,770, and US Pat No. 5,969,095, the entire contents of each of which are hereby incorporated by reference.

Though the PTHrP(1-34) analogue abaloparatide has a similar name to PTH(1-34) teriparatide (PTH is parathyroid hormone and PTHrP is parathyroid hormone-related protein), PTHrP and PTH are the products of different genes with different amino acid sequences. PTH is a hormone that acts as an endocrine regulator of bone resorption and calcium homeostasis. PTH is generally secreted by parathyroid glands in response to low blood serum calcium levels. The increase in PTH hormone indirectly stimulates the formation of osteoclasts. The stimulation of osteoclasts causes bone resorption increasing blood serum calcium levels. PTH also stimulates bone growth by direct interaction with osteoblasts. The duration and periodicity (intermittent vs. continuous) of exposure to PTH determines whether the effects are catabolic (leading to bone resorption) or anabolic (leading to bone growth). (Silvia et al., Curr. Opin Pharmacol, 2015; 22:41-50). In contrast to PTH, PTHrP(1-34) analogues promote osteoblast formation while suppressing the formation of osteoclasts, leading to increased bone formation and density. (Miao et al. Endocrinology. 2004;145:2046-2053; Miao et al. J Clin Invest 2005;115:2402-11; Martin. J Clin Invest 2005;115:2322-4). It is believed that the difference in activity between the two hormones, PTH and PTHrP, may partly be explained by receptor conformational changes when binding to the PTHR1 receptor. Conformational changes that occur when PTH binds to the PTHR1 receptor are believed to stabilize the PTH-PTHR1 complex, leading to longer activation of the G protein and persistent cAMP generation. Persistent cAMP generation leads to more bone resorption. In contrast, PTHrP, and PTHrP analogs only lead to the transient production of cAMP and, therefore, results in less bone resorption. (Tella et al. Cureus 9(5): e1300).

In clinical use, although teriparatide can exhibit rapid and marked increases in bone mineral density at largely trabecular sites (e.g., spine), it also increases cortical porosity. Thus, at sites with a higher proportion of cortical bone, such as the hip or distal forearm, the net effect can be either no change in bone mass or even accelerated bone loss, especially early in treatment, and teriparatide is insufficient to fully reverse bone loss in most patients with severe osteoporosis. In addition, side effects such as hypercalcemia (the presence of too much calcium in the blood) are observed even at a low daily dose of 20 µg per day of teriparatide.

In contrast, abaloparatide increases bone mineral density by stimulating new bone formation through the growth of trabecular bone without compromising cortical bone and with lower associated risk of inducing hypercalcemia as a side effect even at very high doses (e.g. 80 µg sc per day).

Denosumab is a fully human monoclonal IgG antibody with affinity and specificity for human receptor activator of nuclear factor kappa-B ligand (RANKL). Denosumab consists of 2 heavy chains of 448 amino acids, and 2 light chains of 215 amino acids, and includes 36 cysteine residues, which form inter- and intra-chain disulfide bonds. Denosumab and methods relating thereto are described in US Pat. Nos., 6,740,522; 7,411,050; 7,097,834; and 7,364,736, the entire contents of each of which are hereby incorporated by reference.

An effective amount can be achieved in the methods or compositions of the invention by coadministering a first amount of abaloparatide or a pharmaceutically acceptable salt, hydrate or solvate thereof and a second amount of denosumab. In one embodiment, abaloparatide and denosumab are each administered in a respective effective amount (i.e., each in an amount which would be therapeutically effective if administered alone). In another embodiment, abaloparatide and denosumab are each administered in an amount which alone does not provide a sufficient therapeutic effect (a sub-therapeutic dose) when used as a monotherapy. In yet another embodiment, abaloparatide can be administered in an effective amount, while denosumab is administered in a sub-therapeutic dose. In still another embodiment, abaloparatide can be administered in a sub-therapeutic dose, while denosumab is administered in an effective amount. In still another embodiment, both abaloparatide and denosumab can be administered in sub-therapeutic doses.

In certain embodiments, a combination of abaloparatide with denosumab exhibits enhanced therapeutic effect or synergy compared to either abaloparatide or denosumab alone. As used herein, the terms "synergy," "synergism," and "synergistic" relate to the coordinated action of two or more agents with a more than expected additive effect; i.e., the drugs interact in a positive way, making the combination more effective than would be predicted from the activities of the single agents. A synergistic effect may allow a reduction in the effective dosage of one or both of the therapeutic agents. A synergistic effect may, for example, be indicated by an unexpected increase in bone mass density, e.g., in the hip, the spine, or both.

The presence of a synergistic effect can be determined using suitable methods for assessing drug interaction. Suitable methods include, for example, the Sigmoid-Emax equation (Holford, N. H. G. and Scheiner, L. B., Clin. Pharmacokinet. 6: 429-453 (1981)), the equation of Loewe additivity (Loewe, S. and Muischnek, H., Arch. Exp. Pathol Pharmacol. 114: 313-326 (1926)) and the median-effect equation (Chou, T. C. and Talalay, P., Adv. Enzyme Regul. 22: 27-55 (1984)). Each equation referred to above can be applied with experimental data to generate a corresponding graph to aid in assessing the effects of the drug combination. The corresponding graphs associated with the equations referred to above are the concentration-effect curve, isobologram curve and combination index curve, respectively.

In some embodiments, the administration of abaloparatide with denosumab provides an enhanced therapeutic effect. As used herein, "an enhanced therapeutic effect" includes an improved therapeutic profile other than synergy. Examples of enhanced therapeutic effects include, but are not limited to: lowered effective dose of abaloparatide; prolonged therapeutic window of an abaloparatide; minimized toxic side effects of either therapeutic agent; improved ease of administration or use; and/or reduced overall expense of compound preparation or formulation.

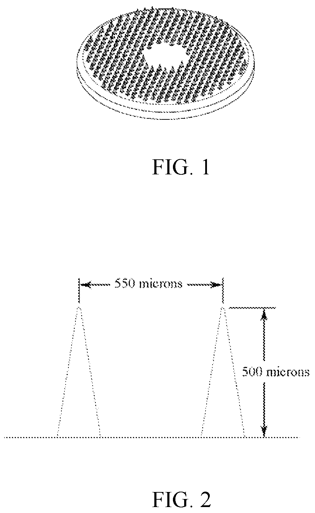

Abaloparatide may be administered by subcutaneous injection (e.g., as described in Int. Publ. No. WO 2008/063279). Alternatively, abaloparatide may be administered transdermally by microprojection or microneedle arrays coated with abaloparatide as described and shown in International Publication Nos. WO 2012/145665, WO2017/062727, WO2017/062922, WO2017/184355, and WO2020/174443 incorporated by this reference in their entirety. Also incorporated is US Patent No. 10,695,289, in its entirety, including but not limited to, Figures 1 and 2 and the accompanying description of a patch microneedle device. An applicator may be employed as described in International Publication No. WO2019/077519, incorporated by this reference in its entirety. Also incorporated by this reference is US Patent No. 10,568,937 in its entirety, including but not limited to Figure 43 and the description of microprojection patches. One patch suitable for use with the disclosed methods is depicted in **FIG. 1** and **FIG. 2****.**

For example, abaloparatide is available as 50 µg, 100 µg, or 150 µg transdermal patches prepared as described in Int. Publ. No. WO 2012/145665 or Int. Publ. No. WO 2013/082427 comprising polycarbonate ("PC") or liquid crystal polymer ("LCP") microneedle arrays. An image of an LCP microarray is set forth in **FIG. 1** and a side view with dimension of the microstructures is set forth in **FIG. 2****.** The abaloparatide-coated PC or LCP microneedle array can be enclosed in a collar assembly for loading onto a spring loaded applicator. The abaloparatide PC or LCP microneedle array is removed from refrigeration 1 hour prior to application and loaded onto the applicator for dosing. In some embodiments, abaloparatide is dosed at 100 µg administered transdermally once daily for clinically appropriate periods.

Suitable doses of abaloparatide include from about 20 to about 400 µg, about 40 to about 300 µg, about 60 to about 200 µg; or from about 80 to about 150 µg, about 20 to about 80 µg, about 20 to about 60 µg, about 20 to about 40 g, about 40 to about 80 µg, about 60 to about 80 µg, about 80 to about 120 µg, about 80 to about 100 µg, about 120 to about 180 µg, about 130 to about 170 µg, about 140 to about 150 µg, about 150 to about 160 µg; or from 40 to about 45 µg, about 45 to about 50 µg, about 50 to about 55 µg, about 55 to about 60 µg, about 60 to about 65 µg, about 65 to about 70 µg, about 70 to about 75 µg, about 75 to about 80 µg, about 80 to about 85 µg, about 85 to about 90 µg, about 90 to about 95 µg, about 95 to about 100 µg, about 100 to about 105 µg, about 105 to about 110 µg, about 110 to about 115 µg, about 115 to about 120 µg, about 120 to about 125 µg, about 125 to about 130 µg, about 130 to about 135 µg, about 135 to about 140 µg, about 140 to about 145 µg, about 145 to about 150 µg, about 150 to about 155 µg, about 155 to about 160 µg administered twice per day, once per day, once every other day, twice per week once per week, once every two weeks, once per month.

Effective doses of abaloparatide can depend on the method of administration, e.g., subcutaneous injection or transdermal delivery.

Suitable effective doses for subcutaneous injection range from 20 to 160 µg administered once per day, once every other day, twice per week, once per week, once every two weeks, or once per month.

In certain embodiments, an effective subcutaneous dose of abaloparatide contains 20 µg, 40 µg, or 80 µg of abaloparatide. In one embodiment an effective subcutaneous dose of abaloparatide is 80 µg of abaloparatide.

In some embodiments, 80 µg of abaloparatide is administered subcutaneously once per day, once every other day, twice per week once per week, once every two weeks, or once per month, for clinically appropriate periods. In some embodiments, 80 µg of abaloparatide is administered subcutaneously once daily. For example, for subcutaneous injection, abaloparatide is available in multi-dose cartridges containing 2 mg/mL abaloparatide (free base) in 5 mg/mL trihydrate sodium acetate and 5 mg/mL of phenol adjusted at pH 5.1 with acetic acid. The multi-dose cartridge is designed to deliver a dose of 80 µg of abaloparatide in 40 mL of fluid when inserted into a pen injector device (BD Pen II). The 80 µg cartridge is removed from refrigeration 1 hour prior to application, and administered into the periumbilical region in a single subcutaneous ("SC") injection.

Suitable effective doses for transdermal delivery range from 50 to 400 µg of abaloparatide administered once, twice, or three times daily. In particular embodiments, effective doses of abaloparatide suitable from transdermal delivery range from 50 to 175 µg.

In some embodiments, abaloparatide is dosed at 300 µg administered transdermally once daily for clinically appropriate periods. In one embodiment, abaloparatide may be administered transdermally as described and shown in International Publication Nos. WO2017/062727, WO2017/062922, WO2017/184355, and WO2020/174443 incorporated by this reference in their entirety. An applicator may be employed to administer as described in International Publication No. WO2019/077519, incorporated by this reference in its entirety. An exemplary circular patch suitable for use in the methods provided herein is depicted in **FIG. 1****,** and a side view of exemplary microstructures is set forth in **FIG. 2****.** The abaloparatide-coated array can be enclosed in a collar assembly for loading onto a spring loaded applicator.

For transdermal application, the microprojection or microneedle arrays may be left in place with one or more microprojections embedded in the subject's skin for a period between 3 seconds and 1 hour (the "wear time"). In certain embodiments, the wear time is from 10 minutes to 30 minutes, or 10 minutes, or 15 minutes. In some embodiments, the wear time is from 3 seconds to 10 minutes, 3 seconds to 5 minutes, or 5 seconds to 3 minutes. In particular embodiments, the wear time is about 5 minutes. In other embodiments, the wear time is 5 seconds, 10 seconds, 15 seconds, 30 seconds, 1 minute, 5 minutes, 10 minutes, 15 minutes or 30 minutes. In one embodiment the wear time is 5 minutes. In another embodiment the wear time is 10 minutes or 15 minutes.

Exemplary dosing schedules, application sites, and wear times are provided in Table 1.

**Table 1: Representative Dosing Schedules for Administration of Abaloparatide Microneedle arrays:**

| **Dosing Frequency** | **Dose** | **Application Site** | **WearTime** |
|---|---|---|---|
| Once | 100 µ g | Periumbilical | 10 seconds |
| Once | 100 µ g | Periumbilical | 5 minutes |
| Once | 100 µ g | Periumbilical | 15 minutes |
| Once | 100 µ g | Upper Thigh | 10 seconds |
| Once | 100 µ g | Upper Thigh | 5 minutes |
| Once | 100 µ g | Upper Thigh | 15 minutes |
| Daily X 7 | 150 µ g | Periumbilical | 10 seconds |
| Daily X 7 | 150 µ g | Periumbilical | 15 minutes |
| Daily X 7 | 150 µ g | Periumbilical | 5 minutes |
| Daily X 7 | 100 µ g | Upper thigh | 5 minutes |
| Daily X 7 | 100 µ g | Upper thigh | 15 minutes |
| Daily X 7 | 150 µ g | Upper Thigh | 5 minutes on Days 1 - 6, 30 seconds on Day 7 |
| Daily X 7 | 150 µ g | Upper Thigh | 1 minute on Days 1 - 6, 60 minutes on Day 7 |
| Daily X 7 | 150 µ g | Upper Thigh | Day 1 24 hours, 15 minutes on Day 7. |

Denosumab is available in pharmaceutical compositions for subcutaneous injection with the brand names PROLIA and XGEVA. XGEVA is supplied as 120 mg/1.7 mL (70 mg/mL) single-use vials and PROLIA is supplied as 1 mL of a 60 mg/mL solution in a single-use prefilled syringe or single-use vial. Denosumab can be administered as 120 mg doses every four weeks by subcutaneous injection in the upper arm, upper thigh, or abdomen, or as 60 mg doses every six months by subcutaneous injection in the upper arm, upper thigh, or abdomen. In some cases, denosumab is administered subcutaneously in 120 mg doses every four weeks in the upper arm, upper thigh, or abdomen with additional 120 mg doses on Days 8 and 15 of the first month of therapy.

Other suitable dosages and dosing schedules of denosumab include from 5 to 30 mg administered subcutaneously every 3 months, for example, 6, 14, or 30 mg subcutaneously every 3 months; or from 10 to 250 mg subcutaneously every 6 months, for example, 14, 60, 100, or 210 mg subcutaneously every 6 months. Additional suitable doses of denosumab include single subcutaneous administration of from 0.01 to 5.0 mg/kg, for example, 0.01, 0.03, 0.1, 0.3, 1.0 or 3.0 mg/kg.

In one embodiment, 80 µg of abaloparatide is administered subcutaneously once per day in combination with denosumab administered every six months for a period of 18 months to a patient with osteoporosis resulting in substantially greater increases in BMD at the spine and hip than achieved by monotherapy with either agent alone.

In one embodiment, 80 µg of abaloparatide is administered subcutaneously once every other day in combination with denosumab administered every six months for a period of 18 months to a patient with osteoporosis resulting in substantially greater increases in BMD at the spine and hip than achieved by monotherapy with either agent alone.

In another embodiment, 80 µg of abaloparatide is administered subcutaneously weekly, twice weekly, three times weekly, every other day, or every two weeks in combination with denosumab administered every six months for a period of 18 months to a patient with mild, moderate or severe osteoporosis resulting in substantially greater increases in BMD at the spine and hip than achieved by monotherapy with either agent alone.

In another embodiment, 80 µg of abaloparatide is administered transdermally weekly, twice weekly, three times weekly, every other day or every two weeks in combination with denosumab administered every six months for a period of 18 months to a patient with mild, moderate or severe osteoporosis resulting in substantially greater increases in BMD at the spine and hip than achieved by monotherapy with either agent alone.

In one embodiment, 20, 40, or 80 µg of abaloparatide is administered subcutaneously once per day in combination with denosumab administered every six months for a period of 18 months to a patient with osteoporosis.

In another embodiment, 80 µg of abaloparatide is administered subcutaneously weekly, twice weekly, three times weekly, every two weeks, or once a month in combination with denosumab administered every six months for a period of 18 months to a patient with osteoporosis.

In another embodiment, 300 µg of abaloparatide is administered transdermally daily in combination with denosumab administered every six months for a period of 18 months to a patient with mild, moderate or severe osteoporosis resulting in substantially greater increases in BMD at the spine and hip than achieved by monotherapy with either agent alone.

In another embodiment, 50 to 400 µg of abaloparatide is administered transdermally once, twice or three times daily in combination with denosumab administered every six months for a period of 18 months to a patient with osteoporosis.

In one embodiment, 80 µg of abaloparatide is administered subcutaneously once per day in combination with 60 mg of denosumab administered every six months for a period of 18 months to a patient with osteoporosis resulting in substantially greater increases in BMD at the spine and hip than achieved by monotherapy with either agent alone.

In another embodiment, 50 to 400 µg of abaloparatide is administered transdermally once, twice or three times daily in combination with 60 mg of denosumab administered every six months for a period of 18 months to a patient with osteoporosis.

In some embodiments, 60 mg of denosumab is administered once at the onset of therapy and abaloparatide is administered daily, weekly, twice weekly, three times weekly, every other day or every two weeks for a period of 18 months.

In some embodiments, 60 mg of denosumab is administered once at the onset of therapy and 50 to 400 µg of abaloparatide is administered transdermally daily, weekly, twice weekly, three times weekly, every other day or every two weeks for a period of 18 months.

In some embodiments, 60 mg of denosumab is administered once at the onset of therapy and 20 to 80 µg of abaloparatide is administered subcutaneously daily, weekly, twice weekly, three times weekly, every other day or every two weeks for a period of 18 months.

In some embodiments, combination therapy may be performed by simultaneous treatment with abaloparatide and denosumab. For simultaneous administration of abaloparatide and detiostimab, denosumab and abaloparatide are administered at the onset of treatment, with abaloparatide treatment being initiated at the time of the first dose of denosumab, or within a period of one day, two days, one week, two weeks, three weeks, or one month of the first dose of denosumab. As used herein the phrase "within a time period" may, for example, refer to a time period that can extend before and/or after the reference point. For example, administering an active agent within a period of one day of an event, should be understood to mean that the active agent is administered at any time that is 1 day before the event and 1 day after the event. Both denosumab and abaloparatide are administered according to their usual dosing schedule. For example, abaloparatide may be administered daily while denosumab is administered every six months.

Alternatively, abaloparatide is administered at the onset of treatment and denosumab treatment is initiated at the time of the first dose of abaloparatide, or within one day, two days, one week, two weeks, three weeks, or one month of the first dose of abaloparatide. Both denosumab and abaloparatide are administered according to their usual dosing schedule. For example, abaloparatide may be administered daily while denosumab is administered every six months.

In some embodiments, combination therapy may be performed sequentially, with denosumab treatment being performed initially, followed by abaloparatide treatment. For sequential combination treatment, abaloparatide is administered to the subject after a predetermined period of time of at least 6 months, at least 12 months, at least 18 months, or at least 24 months after the initial treatment with denosumab. In some embodiments, combination therapy may be performed sequentially, with denosumab treatment being performed initially, followed by abaloparatide treatment no later than 6 months after a treatment with denosumab.

Antiresorptive agents, such as denosumab, can suppress the formation of osteoclasts, reducing the amount of bone resorption. It is believed that denosumab competitively binds to RANKL, inhibiting RANKL from binding to RANK and therefore interfering with the signaling path for the formation of osteoclasts. Initially, treatment with antiresorptive agents lead to increased BMD by inhibiting bone resorption. The inhibition of bone resorption, however, is typically followed by an eventual decrease in osteoblast activity. After prolonged denosumab treatment, BMD may stop increasing, and may eventually start decreasing, even while denosumab treatment is continued. The terms "refractory osteoporosis" and "denosumab-refractory osteoporosis" refer to osteoporosis that is not adequately treated by denosumab such that the subject remains at high risk for fracture, bone mass density (BMD) declines, or BMD remains persistently low, despite continued treatment.

In one embodiment, denosumab-refractory osteoporosis is treated by administering abaloparatide. Abaloparatide acts as an anabolic agent that promotes bone growth, counteracting the loss of bone growth encountered after extended use of denosumab. Abaloparatide upregulates RANKL and reduces OPG, increasing the RANKL:OPG ratio. (Makino, CTI 2018)

In some embodiments, denosumab is administered at the onset of treatment. When denosumab-refractory osteoporosis is observed, abaloparatide treatment is initiated. In an embodiment, abaloparatide is administered in combination with denosumab treatment. In another embodiment, denosumab treatment is discontinued when abaloparatide treatment is initiated. In another embodiment, after a period of 6 months, 9 months, 12 months, 15 months, 18 months, 21 months, or 24 months of denosumab treatment, abaloparatide treatment is automatically initiated. Both denosumab and abaloparatide are administered according to their usual dosing schedule. For example, abaloparatide may be administered daily while denosumab is administered every six months. In some embodiments, abaloparatide is initiated no later than 6 months after a previous administration of denosumab, and denosumab treatment can be ongoing or terminated.

In some embodiments, it is necessary, due to side effects caused by continued usage of denosumab, to discontinue treatment with denosumab. Discontinuation of the use of denosumab is associated with a sudden loss of BMD due to an increase in production of osteoclasts. Abaloparatide may be used to minimize bone loss after discontinuation of denosumab therapy. In an embodiment, abaloparatide treatment may be initiated within one month, within two months, within three months, within 6 months, within 9 months or within 12 months after treatment with denosumab is discontinued. In other embodiments, abaloparatide treatment may be initiated before treatment of denosumab is discontinued. In an embodiment, abaloparatide treatment is initiated within three months, within two months, within one month, within three weeks, within two weeks, or within one week of the anticipated last dose of denosumab.

In some embodiments, combination therapy may be performed sequentially, with abaloparatide treatment being performed initially, without concomitant denosumab treatment. Denosumab may later be administered to the subject. In an embodiment, denosumab is initiated at least one month, at least two months, at least three months, at least 6 months, at least 9 months or at least 12 months after treatment with abaloparatide has started. In an alternate embodiment, the BMD of a subject may be monitored while abaloparatide is being administered. Treatment with denosumab, in combination with abaloparatide, may be initiated when the BMD of the subject is at or below the mean BMD for a representative population after treatment.

Also provided is a kit for prevention or treatment of osteoporosis, osteopenia, osteoarthritis or bone fracture; to accelerate bone fracture healing; or to increase bone mineral density. The kit comprises a first compound that is a PTHrP analogue (e.g., abaloparatide), a second compound that is denosumab and instructions for administering the first and second compounds, and optionally a device for administering the compounds.

Further embodiments of the invention are provided herein (as non-limiting examples):
1. A method of increasing bone mineral density in a subject in need thereof comprising administering to said subject:
   a a first amount of [Glu22,25, Leu23,28,31, Aib29, Lys26,30]hPTHrP(1-34)NH2 or a pharmaceutically acceptable salt thereof, and
   b. a second amount of denosumab,
      wherein the first and second amounts together comprise an effective amount.
2. A method of treating osteoporosis in a subject in need thereof comprising administering to said subject:
   a a first amount of [Glu22,25, Leu23,28,31, Aib29, Lys26,30]hPTHrP(1-34)NH2 or a pharmaceutically acceptable salt thereof, and
   b. a second amount of denosumab,
      wherein the first and second amounts together comprise an effective amount.

The aspects and embodiments of the invention disclosed herein may be combined. In particular, the various possible combinations of features are specifically envisaged and disclosed, including the possibility of combining features from various aspects and embodiments, e.g. from different sections of the specification.

### Definitions

The term "abaloparatide" refers to [Glu^{22,25}, Leu^{23,28,31}, Aib²⁹, Lys^{26,30}]hPTHrP(1-34)NH₂ (SEQ ID NO.: 1). Abaloparatide is also known as BA058.

As used herein, the term "subject" refers to a mammal, preferably a human, but can also mean an animal in need of veterinary treatment, e.g., companion animals (e.g., dogs, cats, and the like), farm animals (e.g., cows, sheep, pigs, horses, and the like) and laboratory animals (e.g., rats, mice, guinea pigs, and the like).

As used herein, the term "effective amount" or "effective dose" or "therapeutically effective amount" refers to an amount that, when administered in a proper dosing regimen, is sufficient to treat the target disorder or elicit the desired biological response. The desired biological response may be a decrease in the rate of bone loss and/or an increase in the bone mass or bone quality of a subject. The minimum effective dose (MED) is the lower limit of the therapeutic window; the upper limit of the therapeutic window is the maximum tolerated dose (MTD).

The term "treating" or "treatment" may refer to, e.g., treating, preventing, or ameliorating the symptoms associated with, or reducing the incidence of, reducing the pathogenesis of, facilitating the recovery from or delaying the onset of the condition being considered including osteopenia, osteoporosis, osteoarthritis, bone fracture, and so forth. The term "preventing" may refer to preventing or delaying the disease or symptom from occurring in a subject which may be predisposed to the disease or symptom but has not yet been diagnosed as having it.

The term "treating" or "treatment" may alternatively refer to treatment of a mammal, preferably a human, and understood to include treating, preventing, or ameliorating the symptoms associated with, or reducing the incidence of, reducing the pathogenesis of, facilitating the recovery from or delaying the onset of the condition being considered including osteopenia, osteoporosis, osteoarthritis, bone fracture, and so forth. The methods and combinations in accordance with the invention can be demonstrated in various animal models known in the art, including, for example, an ovariectomized (OVX) primate or rabbit model.

The term "preventing" as used herein is understood to mean preventing or delaying the disease or symptom from occurring in a subject which may be predisposed to the disease or symptom but has not yet been diagnosed as having it.

As used herein, the unit microgram may be represented by either "mcg" or "µg".

The term "adverse event" or "AE" may, for example, refer to: any untoward or unfavorable medical occurrence in a human subject, including any abnormal sign (for example, abnormal physical exam or laboratory finding), symptom, or disease, temporally associated with the subject's participation in the research, whether or not considered related to the subject's participation in the research. An AE does not necessarily have a causal relationship with the treatment or study.

The term "serious adverse event" may, for example, refer to an AE that results in death, is life threatening, results in inpatient hospitalization or prolongation of existing hospitalization, or results in a persistent or significant disability/incapacity.

The term "adverse reaction" may, for example, mean any adverse event caused by a drug.

The term "associated with one another" as used herein may, for example, mean that the separate dosage forms are packaged together or otherwise attached to one another such that it is readily apparent that the separate dosage forms are intended to be sold and administered together (within less than 24 hours of one another, consecutively or simultaneously) for the prevention or treatment of osteoporosis, osteopenia, osteoporosis, osteoarthritis, or bone fracture; to accelerate bone fracture healing; or to increase bone mineral density.

Aspects of the present teachings can be further understood in light of the following examples, which should not be construed as limiting the scope of the present teachings in any way. Based on the activity shown in the standard pharmacological test procedures, the methods and combinations of the present teachings can be useful for the prevention or treatment of osteoporosis, osteopenia, osteoarthritis, or bone fracture; to accelerate bone fracture healing; to increase bone mineral density; or to reduce the risk of future fractures in a subject in need thereof.

Some methods suitable to demonstrate preclinical efficacy of treatments for osteoporosis are known in the art, and include, for example, the Guidelines for Preclinical and Clinical Evaluation of Agents used in the Prevention or Treatment of Postmenopausal Osteoporosis published by the Division of Metabolism and Endocrine Drug Products, Food and Drug Administration, U.S.A. Methods for the clinical evaluation of treatments for osteoporosis are known, and include, for example, "Recommendations for the Clinical Evaluation of Agents for Treatment of Osteoporosis: Consensus of an Expert Panel Representing the American Society for Bone and Mineral Research (ASBMR), the International Society for Clinical Densitometry (ISCD), and the National Osteoporosis Foundation (NOF)," Journal of Bone and Mineral Research, 23 (2008), 159-165.

### EXEMPLIFICATION (Evaluation of changes in bone mineral density and bone microstructure)

### Example 1: Abaloparatide and Denosumab Therapy in OVX Cynomolgus Monkey Model

The effect of [Glu^{22,25}, Leu^{23,28,31}, Aib²⁹, Lys26,30]hPTHrP(1-34)NH₂ (abaloparatide, ABL) and denosumab can be assessed in an aged osteopenic ovariectomized (OVX) cynomolgus monkey bone loss model (for example, as in Smith et al, . Bone, 57, (2013), 116-122). Efficacy is assessed by the effect of test articles on bone mineral density (BMD) measured by densitometry techniques such as dual energy x-ray absorptiometry (DXA) and peripheral quantitative computed tomography (pQCT); bone micro-structure as assessed by micro-computed tomography (microCT), and urine and serum bone turnover markers. Bone markers and DXA/pQCT are measured prior to OVX/sham surgery, end of bone depletion, at regular (e.g, every 1-3 months) intervals during treatment, and at the end of study.

Mauritius female cynomolgous monkeys ≥ 9 years of age undergo either ovariectomy or sham ovariectomy surgery followed by a 9-month bone depletion period. Dosing can be performed for periods ranging from 14 days to up to 18 months. Following completion of baseline bone mineral density (BMD) measurements by dual-energy X-ray absorptiometry (DXA), animals are randomly assigned to groups and groups balanced to ensure that age, body weight, whole body bone mineral content (BMC), and lumbar spine BMD are equivalent across groups.

For all animals, the percent change in BMD from the end of the bone depletion period to end of study is assessed by DXA. The images are analyzed using the provided software to determine the BMD of the lumbar spine (L1-L4), thoracic spine (T9-T12), and proximal femoral neck. Volumetric bone mineral content (vBMC) and volumetric BMD (vBMD) at metaphyseal and diaphyseal sites of the right tibia is measured by pQCT at baseline and at defined monthly intervals during treatment. Metaphyseal data are generated as an average from 3 scans separated by 0.5 mm at the tibia/fibula junction, and a diaphyseal scan is taken at approximately 12% of the bone length toward the center of the tibia from the metaphyseal scans.

Trabecular parameters such as bone volume density (BV/TV), trabecular number (Tb.N), trabecular thickness (Tb.Th), trabecular spacing (Tb.Sp), and apparent bone density (ABD) are assessed by bone histomorpometric analysis.

Exemplary bone turnover markers include serum bone specific alkaline phosphatase (BAP), urinary collagen type 1 cross-linked N-telopeptide (NTX), and serum collagen type 1 cross-linked C-telopeptide (CTX).

Doses of abaloparatide suitable for use in cynomolgous monkeys range from about 0.2 to about 5 µg/kg (e.g., 0.2 1, or 5 µg/kg) for subcutaneous injection and from about 10 to about 75 mcg for transdermal administration.

Doses of denosumab suitable for use in cynomolgous monkeys range from about 5 to about 100 mg/kg (e.g., 25 or 50 mg/kg).

Representative study treatment groups for the assessment of the combination of abaloparatide subcutaneous injection and denosumab are provided in Table 2 (provided as non-limiting examples):

**Table 2: Representative study treatment groups**

| **Surgery** | **Dose** (µg) | **Test Group** |
|---|---|---|
| SHAM | 0 (Placebo) | Vehicle control |
| OVX | 0 (Placebo) | Vehicle control |
| OVX | ABL (sc qd) | Bone optimal dose |
| OVX | DEN (sc qd) | Bone optimal dose |
| OVX | ABL (sc qd)+ DEN (sc qd) | ABL bone optimal dose + DEN bone optimal dose |
| OVX | ABL (sc qd)+ DEN (sc qd) | ABL bone optimal dose + DEN bone suboptimal dose |
| OVX | ABL (sc qd)+ DEN (sc qd) | ABL bone suboptimal dose +DEN bone optimal dose |
| OVX | ABL (sc qd)+ DEN (sc qd) | ABL bone suboptimal dose +DEN bone suboptimal dose |
| OVX = ovariectomy; ABL = Abaloparatide; DEN = denosumab; sc = subcutaneous, qd = every day | | |

Abaloparatide can be administered by either abaloparatide-PC-microneedle arrays, abaloparatide-LCP-microneedle arrays, or abaloparatide subcutaneous injection. The appropriate placebo form, either placebo microneedle arrays or placebo subcutaneous injection, is used for vehicle control groups. Microarrays are left in contact with the skin for the prescribed time (for example, for between 10 seconds and 30 minutes) before being removed.

As used herein, "bone optimal dose" is an amount which produces the greatest therapeutic effect while still being associated with acceptable safety and tolerability. A "bone suboptimal dose" is an amount which produces a substantially reduced therapeutic effect compared to the "bone optimal dose". The combination of a suboptimal dose of one agent and an optimal dose of another agent, or of suboptimal doses of both agents, may together comprise an effective amount.

***Results.*** It is believed that this study will demonstrate the suitability of the combination of abaloparatide and denosumab for the methods of the invention, for the reasons provided above. It is additionally believed that the combination of abaloparatide and denosumab will demonstrate efficacy in this model for the reasons explained above. For example, it is expected that abaloparatide with continued denosumab treatment will allow bone formation to increase, without increasing bone resorption and will produce substantial BMD increments, e.g. in both spine and hip. It is anticipated that in animal subjects who are at high risk for fracture while receiving ongoing denosumab therapy, adding abaloparatide will increase BMD significantly more than continuing denosumab alone, e.g. of the lumbar spine, thoracic spine, proximal femoral neck, and hip.

### Example 2: Treatment with abaloparatide added to ongoing denosumab in humans

This randomized open label clinical trial will evaluate the effect of continued denosumab alone over 18 months versus denosumab with added abaloparatide for 18 months. 70 postmenopausal women will be enrolled over a period of 18 months. The co-primary outcomes will be group differences in BMD of the total hip and lumbar spine at 18 months. Secondary outcomes will include group differences in BMD at the femoral neck, trochanter and wrist sites at 6, 12 and 18 months, spine and total hip BMD at 6 and 12 months and TBS at 18 months. Secondary outcomes will also include within group changes from baseline for each of these variables. Bone turnover markers will also be measured to demonstrate that PINP levels will increase with administration of abaloparatide even in the setting of ongoing denosumab, while CTX levels will remain low.

***Inclusion Criteia.*** Subjects will be postmenopausal >age 45 of any racial origin (with postmenopausal status defined as no menses for one year and confirmed by gynecologist investigator, Dr. Jacobson). Participants will have received at least 4 prior denosumab treatments and be within 7 months from their last denosumab injection upon enrolling in the trial. They should be willing to participate for the duration of the study and have no physical or psychological illness that would prohibit them from participating. Participants will have a diagnosis of osteoporosis based on BMD and/or fracture criteria. Osteoporosis will be defined by BMD T-Score < -2.5 at lumbar spine (at least 2 evaluable vertebrae between L1 and L4), total hip or femoral neck. Osteoporosis will also be defined clinically in women with osteoporotic fractures within the preceding 5 years, including clinical vertebral or nonvertebral fractures or vertebral fracture confirmed by radiograph or lateral DXA VFA image, along with a DXA BMD T-Score < -1.5 at one or more skeletal sites.

***Exclusion Criteria.*** Excluded from this study will be subject who have used medications to treat osteoporosis other than denosumab. Past use of bisphosphonates (prior to denosumab treatment) is allowable, as is low dose estrogen for control of menopausal symptoms, and use of SERMs. Also excluded are subjects with any of the following exclusion criteria: use of glucocorticoids for more than 2 weeks in the past 3 months; current use of aromatase inhibitors; prior use of teriparatide or abaloparatide for more than 6 months; fewer than 2 evaluable lumbar vertebrae; bilateral total hip replacements; a history of a symptomatic renal stone within the past 2 years or history of multiple symptomatic renal stones within the preceding 5 years; skeletal Disorders other than osteoporosis, including hypercalcemia, hyperparathyroidism, or Paget's Disease; a history of external or internal radiation therapy; serum 25OHD level <25 ng/ml; estimated GFR below 30 ml/min; any contraindications to receipt of abaloparatide or denosumab; a history of any cancer in past 5 years (except basal/squamous skin cancer); unexplained elevation of Serum Alkaline Phosphatase; a history of an atypical femur fracture; and disorders of immunosuppression or use of anti-angiogenic medications.

***Screening visit.*** At the screening visit, a comprehensive medical history and brief physical exam will be performed. Blood samples will be obtained and serum levels of calcium, intact-parathyroid hormone, 25(OH)D, creatinine and alkaline phosphatase will be measured. BMD of the spine, hip and radius will be measured by DXA and VFA will be performed to diagnose vertebral fractures. Subjects who meet inclusion and exclusion criteria will be enrolled and present for a baseline visit.

***Baseline Visit:*** Subjects will present to the research nurse in the morning after an overnight fast. Dietary calcium and vitamin D intakes will be ascertained. Dietary changes and calcium supplements (if needed) will be recommended to bring total calcium intake to 1200 mg daily. Vitamin D supplements will be recommended to attain and maintain serum 250HD levels above approximately 30 ng/ml. Baseline blood samples for biochemical indices of bone turnover (serum Propeptide of type I procollagen, PINP, for bone formation and serum C-telopeptide, CTX, for bone resorption) will be obtained. (For patients who enroll in the study more than 3 months from their screening DXA, BMD measurements will be repeated. Otherwise, the screening DXA will serve as the baseline measurement). Trabecular Bone Score (TBS) will also be assessed based on screening/baseline DXA.

***Treatment assignment and randomization:*** The study will be randomized but open label. Once volunteers are enrolled, the research associate will request group allocation. Subjects will be randomized in a 1:1 ratio to one of two treatment arms: (1) Denosumab alone: 3 injections of Denosumab at appropriate times, separated by no more than 7 months from the last treatment; or (2) Combination therapy: Denosumab as above, with added abaloparatide 80 mcg subcutaneously daily, started within 6 months of the last denosumab treatment, for a total of 18 months.

***Study visits:*** Subjects will be seen by the research nurse on-site at 3, 6, 12 and 18 months. At all visits, interval histories will be taken and will include assessment of fractures, falls, back pain, intercurrent illness, concomitant medications, compliance and adverse events/side effects. BMD will be measured at spine, hip and 1/3 radius by DXA every 6 months. TBS will be analyzed at baseline and 18 months and VFA will be analyzed at screening and at 18 months.

Fasting AM blood samples will be obtained at each study visit (B and months 3, 6, 12 and 18) and biochemical turnover markers (serum PINP and CTX) will be measured. All bone turnover samples will be centrifuged, serum separated into 0.5 ml aliquots and stored in a -70-degree freezer at the Crozer-Keystone Osteoporosis Center.

Denosumab will be administered every 6 months to all participants and abaloparatide dispensed per protocol to the abaloparatide plus continued denosumab combination group.

The most common adverse reactions (incidence ≥2%) with abaloparatide are hypercalciuria, dizziness, nausea, headache, palpitations, fatigue, upper abdominal pain and vertigo. At each visit, while on abaloparatide, adverse events (AEs) or serious adverse events (SAEs), comorbidities and concomitant medication use will be recorded. Follow-up for any SAE will occur until it is resolved.

At the end of this study, patients will be advised to either continue denosumab or transition to a bisphosphonate to minimize risk of bone loss and multiple vertebral fractures. This decision will be made by the patient's primary treating physician, based on a reassessment of risks and benefits for each therapeutic option for each individual.

***Statistical analysis.*** Analyses will be performed using SAS software, Version 9.2 (Cary, N.C.). Descriptive statistics will be calculated to characterize the study population at baseline. Differences between the denosumab alone and denosumab plus abaloparatide treatment groups in demographic variables, including age, spine and hip BMD, BMI and bone turnover levels, will be assessed using to-tests for continuous variables and chi-square tests for categorical variables. Non-normally distributed data will be analyzed with the appropriate non-parametric counterpart. Analyses will be done using intention to treat as well as per protocol analysis, which will include subjects who have been randomized, have received at least 80 percent of their abaloparatide doses and all three denosumab injections and who do not have substantial protocol violations. Missing data will be handled by interpolation between the proximate before and after values for BMD and bone turnover.

The co-primary outcomes will be group differences in BMD of the total hip and lumbar spine at 18 months. Secondary outcomes will include group differences in BMD at the femoral neck, trochanter and wrist sites at 6, 12 and 18 months, spine and total hip BMD at 6 and 12 months and TBS at 18 months. Secondary outcomes will also include within group changes from baseline for each of these variables. Bone turnover markers will also be measured to demonstrate that PINP levels will increase with administration of abaloparatide even in the setting of ongoing denosumab, while CTX levels will remain low.

The co-primary endpoints of BMD changes at the total hip and spine will be measured by DXA. Endpoints will also include BMD at the other hip sites and forearm (by DXA) and TBS. Change in BMD over the 18-month period will be analyzed within treatment groups by a paired t-test and between treatment groups by a two-sample t-test with a two-sided error rate of 5%. The primary analyses will be performed using analysis of covariance to control for other variables while determining the treatment effects. For each participant, percentage change will be calculated. This variable will be entered into a multivariate model adjusted for potential confounding variables such as age, BMI, baseline bone density or bone turnover. The effect of treatment groups will be defined as the difference in mean percent change of BMD at each skeletal site between these groups using group differences by repeated measures analysis of variance for BMD.

***Bone turnover markers.*** Means, medians, and standard errors of the mean, will be calculated for each biochemical variable at baseline and during treatment, according to treatment group. To investigate any differences within the treatment groups over time in markers of bone turnover, non-normally distributed variables will be log transformed prior to analysis. Analysis of covariance will be used to evaluate whether these outcome variables change independently of other variables, such as age, and baseline BMD. Repeated measures analysis of variance will be used to assess differences in bone turnover before and, every 6 months after treatment begins, within and between treatment groups to investigate differences in time trends or rates of change among groups.

***Key outcome variables.*** The Co-Primary endpoints are group differences in BMD increment at total hip and lumbar spine at 18 months. The secondary endpoints are: (1) Group Differences in BMD increments of lumbar spine and total hip at 6 and 12 months, and of femoral neck, and 1/3 radius at 6, 12 and 18 months; (2) Group differences in TBS at 18 months; (3) Within Group Increments in BMD (vs baseline) of lumbar spine at 6, 12 and 18 months, of total hip, femoral neck and 1/3 radius at 6, 12 and 18 months, and of TBS at 18 months; and (4) Within and between group differences in biochemical bone turnover markers (PINP and CTX) at 3, 6, 12 and 18 months.

***Results (efficacy).*** This study was posted on July 13, 2020 on ClinicalTrials.gov (clinicaltrials.gov/ct2/show/NCT04467983) and has an expected completion date of August 2022. It is believed that this study will demonstrate the suitability of the combination of abaloparatide and denosumab for the methods of the invention, for the reasons provided above. It is additionally believed that the combination of abaloparatide and denosumab will meet the Co-Primary endpoints, and the secondary endpoints, for the reasons explained above. For example, it is expected that abaloparatide with continued denosumab treatment will allow bone formation to increase, without increasing bone resorption and will produce substantial BMD increments, e.g. in both spine and hip. It is anticipated that in animal subjects who are at high risk for fracture while receiving ongoing denosumab therapy, adding abaloparatide will increase BMD significantly more than continuing denostimal) alone, e.g. of the lumbar spine, thoracic spine, proximal femoral neck, and hip.

***Results (adverse events).*** It is believed that this study will demonstrate a significant reduction in adverse events that are typically observed with abaloparatide and denosumab individually (e.g. hypotensive effects, dizziness, palpitation and nausea side effects). Without wishing to be bound by theory, it is believed that the combination of abaloparatide and denosumab will reduce adverse events by providing a favourable PTH profile since abaloparatide is capable of decreasing endogenous PTH levels while denosumab is capable of increasing PTH levels.

Unless defined otherwise, all technical and scientific terms used herein have the same meaning as commonly understood by one of ordinary skill in the art. All publications, patent applications, patents and other references mentioned herein are incorporated by reference in their entirety.

While this invention has been particularly described with references to example embodiments thereof, it will be understood by those skilled in the art that various changes in form and details may be made therein without departing from the scope of the invention encompassed by the appended claims.
The invention also relates to the following numbered embodiments:
1. A method of increasing bone mineral density in a subject in need thereof comprising administering to said subject:
   a first amount of abaloparatide, and
   a second amount of denosumab,
   wherein bone mineral density is increased in the subject in response to administration of abaloparatide and denosumab.
2. A method of treating osteoporosis in a subject in need thereof comprising administering to said subject:
   a first amount of abaloparatide, and
   a second amount of denosumab,
   wherein the osteoporosis is treated in the subject in response to administration of abaloparatide and denosumab.
3. The method according to embodiment 1 or embodiment 2 wherein the first amount of abaloparatide is 80 mcg daily administered subcutaneously or 300 mcg daily administered transdermally.
4. The method according to any of the preceding embodiments wherein the second amount of denosumab is 60 mg administered subcutaneously every six months.
5. The method according to any of the preceding embodiments where the abaloparatide is administered concomitantly with the administration of denosumab.
6. The method according to any one of embodiments 1-4 where the abaloparatide is administered before administration of denosumab.
7. The method according to any one of embodiments 1-4 where the denosumab is administered before administration of abaloparatide.
8. The method according to any of the preceding embodiments where the total hip BMD is increased at least 10% in subjects where abaloparatide is administered with ongoing treatment with denosumab for 18 months.
9. The method according to any of the preceding embodiments where the total spine BMD is increased at least 15% in subjects where abaloparatide is administered with ongoing treatment with denosumab for 18 months.
10. The method according to any of the preceding embodiments, wherein denosumab and abaloparatide are administered at the onset of treatment, with abaloparatide treatment being initiated at the time of the first dose of denosumab, or within a period of one month of the first dose of denosumab.
11. The method according to any of embodiments 1-9, wherein denosumab is administered at the onset of treatment and abaloparatide treatment is initiated at least 6 months after initial treatment with denosumab.
12. The method according to any of embodiments 1-9, wherein abaloparatide is administered at the onset of treatment and denosumab treatment is initiated at least one month after treatment with abaloparatide is initiated.
13. A method of treating denosumab-refractory osteoporosis, the method comprising:
   administering an effective amount of abaloparatide to a subject having denosumab-refractory osteoporosis,
   wherein administration of an effective amount of abaloparatide to the subject increases bone formation in the subject without increasing bone resorption.
14. The method of embodiment 13, wherein abaloparatide is administered in combination with denosumab treatment.
15. The method of embodiment 13, wherein denosumab treatment is discontinued when abaloparatide treatment is initiated.
16. The method of embodiment 15, wherein abaloparatide treatment is initiated within one month after treatment with denosumab is discontinued.
17. The method of embodiment 13, wherein abaloparatide treatment is initiated within three months of the anticipated last dose of denosumab.
18. The method according to any one of embodiments 13-17, wherein administration of an effective amount of abaloparatide to the subject increases the subject's lumbar spine BMD or hip BMD relative to treatment with denosumab alone.
19. The method according to any one of embodiments 13-18, wherein the total hip BMD is increased at least 10% after 18 months of administration of abaloparatide.
20. The method according to any of the preceding embodiments, wherein the subject is a subject at high risk for a fracture despite denosumab treatment, as indicated by declining or persistently low bone mass density.
21. The method according to any of the preceding embodiments, wherein the subject has had denosumab therapy, followed by teriparatide therapy, and a total hip BMD below the baseline at the start of teriparatide therapy.

## Claims

1. Abaloparatide for use in a method of treating osteoporosis in a subject in need thereof, wherein the method comprises administering to said subject:
abaloparatide in an amount of 80 micrograms per day, administered subcutaneously; and
denosumab in an amount of 60 mg, administered subcutaneously every six months, wherein the abaloparatide is administered concomitantly with the administration of denosumab, and wherein the osteoporosis is treated in the subject in response to administration of abaloparatide and denosumab.

2. The abaloparatide for use according to claim 1, wherein the osteoporosis is denosumab-refractory osteoporosis.

3. The abaloparatide for use according to claim 1 or 2, wherein the subject is at high risk for a fracture despite prior denosumab treatment, wherein the high risk for fracture is determined by a declining or persistently low bone mass density.

4. The abaloparatide for use according to any preceding claim, wherein the subject has had prior denosumab therapy, followed by teriparatide therapy, and a total hip BMD below the baseline at the start of teriparatide therapy.

5. The abaloparatide for use according to any preceding claim, wherein a total hip BMD is increased at least 10% in subjects where abaloparatide is administered with ongoing treatment with denosumab for 18 months.

6. The abaloparatide for use according to any preceding claim, wherein the total spine BMD is increased at least 15% in subjects where abaloparatide is administered with ongoing treatment with denosumab for 18 months.

7. The abaloparatide for use according to claim 2, wherein administration of an effective amount of abaloparatide to the subject increases lumbar spine BMD relative to treatment with denosumab alone.
